# EUROPEAN PATENT SPECIFICATION

(11) **EP 1 697 531 B1**
(45) Date of publication and mention of the grant of the patent: **18.05.2016**
(21) Application number: 04803898.8
(22) Date of filing: 15.12.2004
(51) Int. Cl.: C12P 13/08, C12R 1/19

(54) **PROCESS FOR PREPARING L-AMINO ACIDS USING STRAINS OF THE ENTEROBACTERIACEAE FAMILY**
VERFAHREN ZUR HERSTELLUNG VON L-AMINOSÄUREN UNTER VERWENDUNG VON STÄMMEN DER FAMILIE ENTEROBACTERIACEAE
PROCEDE POUR PREPARER DES ACIDES L-AMINO AU MOYEN DE SOUCHES DE LA FAMILLE DES ENTEROBACTERIES

(30) Priority: 24.12.2003 DE 10361192
(43) Date of publication of application: 06.09.2006
(73) Proprietor: Evonik Degussa GmbH, 45128 Essen (DE)
(72) Inventor: RIEPING, Mechthild, 33619 Bielefeld (DE)
(86) International application number: PCT/EP2004/014279
(87) International publication number: WO 2005/064001

(56) References cited:
- WO-A-03/008605
- WO-A-03/008606
- WO-A-03/008608
- US-A- 4 278 765
- PUSKÁROVÁ A ET AL: "Regulation of yodA encoding a novel cadmium-induced protein in Escherichia coli." MICROBIOLOGY (READING, ENGLAND) DEC 2002, vol. 148, no. Pt 12, December 2002 (2002-12), pages 3801-3811, XP002334861 ISSN: 1350-0872 cited in the application
- DATABASE MEDLINE [Online] US NATIONAL LIBRARY OF MEDICINE (NLM), BETHESDA, MD, US; December 1992 (1992-12), MASUDA M ET AL: "Improvement of nitrogen supply for L-threonine production by a recombinant strain of Serratia marcescens." XP002334866 Database accession no. NLM1363856 & APPLIED BIOCHEMISTRY AND BIOTECHNOLOGY. DEC 1992, vol. 37, no. 3, December 1992 (1992-12), pages 255-265, ISSN: 0273-2289
- DAVID G ET AL: "YodA from Escherichia coli is a Metal-binding, Lipocalin-like Protein" JOURNAL OF BIOLOGICAL CHEMISTRY 31 OCT 2003 UNITED STATES, vol. 278, no. 44, 31 October 2003 (2003-10-31), pages 43728-43735, XP002334862 ISSN: 0021-9258
- FERIANC P ET AL: "The cadmium-stress stimulon of Escherichia coli K-12." MICROBIOLOGY (READING, ENGLAND) APR 1998, vol. 144 ( Pt 4), April 1998 (1998-04), pages 1045-1050, XP002334863 ISSN: 1350-0872

### SEQUENCE LISTING

<110> Degussa AG
<120> Process for preparing L-amino acids using strains of the Enterobacteriaceae family
<130> 020319 BT
<160> 6
<170> PatentIn Version 3.2
<210> 1
   <211> 30
   <212> DNA
   <213> Artificial sequence
<220>
   <221> Primer
   <222> (1)..(30)
   <223> yodA_5'
<220>
   <221> Restriction cleavage site
   <222> (4)..(9)
   <223> NcoI
<400> 1
   ggtccatggc gattcgtctt tacaaactgg 30
<210> 2
   <211> 22
   <212> DNA
   <213> Artificial sequence
<220>
   <221> Primer
   <222> (1)..(22)
   <223> yodA_3'
<220>
   <221> Restriction cleavage site
   <222> (15)..(20)
   <223> HincII
<400> 2
   ggaatccgtt attggttaac tc 22
<210> 3
   <211> 823
   <212> DNA
   <213> Escherichia coli
<220>
   <221> yodA
   <222> (1)..(823)
   <223> yodA PCR product
<220>
   <221> CDS
   <222> (6)..(656)
   <223> yodA (b1973)-orf
<400> 3
   ggtcc atg gcg att cgt ctt tac aaa ctg gct gtt gct tta ggt gtc ttt      50
         Met Ala Ile Arg Leu Tyr Lys Leu Ala Val Ala Leu Gly Val Phe
         1               5                   10                  15
   att gtt agc gct cct gcc ttt tcg cat ggt cat cac tca cac ggc aaa        98
   Ile Val Ser Ala Pro Ala Phe Ser His Gly His His Ser His Gly Lys
                   20                  25                  30
   ccc tta aca gag gtc gaa caa aaa gct gct aat ggt gtt ttt gat gat       146
   Pro Leu Thr Glu Val Glu Gln Lys Ala Ala Asn Gly Val Phe Asp Asp
               35                  40                  45
   gcc aat gta caa aac cga acg ctc agt gac tgg gat gga gtc tgg caa       194
   Ala Asn Val Gln Asn Arg Thr Leu Ser Asp Trp Asp Gly Val Trp Gln
           50                  55                  60
   tcc gtt tat cct tta ctg caa agt ggc aaa ctt gac ccc gtc ttt cag       242
   Ser Val Tyr Pro Leu Leu Gln Ser Gly Lys Leu Asp Pro Val Phe Gln
       65                  70                  75
   aag aaa gcg gat gca gat aaa act aaa aca ttt gct gaa att aaa gat       290
   Lys Lys Ala Asp Ala Asp Lys Thr Lys Thr Phe Ala Glu Ile Lys Asp
   80                  85                  90                  95
   tat tat cac aaa ggt tat gca aca gat atc gag atg att ggc att gag       338
   Tyr Tyr His Lys Gly Tyr Ala Thr Asp Ile Glu Met Ile Gly Ile Glu
                   100                 105                 110
   gac ggc att gtt gaa ttc cat aga aat aat gaa aca aca tcg tgt aaa       386
   Asp Gly Ile Val Glu Phe His Arg Asn Asn Glu Thr Thr Ser Cys Lys
               115                 120                 125
   tat gat tac gat gga tac aaa ata ctc acc tat aaa tca ggc aag aaa       434
   Tyr Asp Tyr Asp Gly Tyr Lys Ile Leu Thr Tyr Lys Ser Gly Lys Lys
           130                 135                 140
   ggc gtt cgt tac tta ttc gaa tgt aaa gat cct gaa agc aaa gcc cct       482
   Gly Val Arg Tyr Leu Phe Glu Cys Lys Asp Pro Glu Ser Lys Ala Pro
       145                 150                 155
   aaa tat ata caa ttt agc gat cat ata att gca cca cga aaa tca tct       530
   Lys Tyr Ile Gln Phe Ser Asp His Ile Ile Ala Pro Arg Lys Ser Ser
   160                 165                 170                 175
   cat ttt cac att ttt atg ggt aat gat tca cag caa tct tta tta aat       578
   His Phe His Ile Phe Met Gly Asn Asp Ser Gln Gln Ser Leu Leu Asn
                   180                 185                 190
   gaa atg gaa aac tgg cca acg tat tat cca tat cag ttg agt agc gaa       626
   Glu Met Glu Asn Trp Pro Thr Tyr Tyr Pro Tyr Gln Leu Ser Ser Glu
               195                 200                 205
   gaa gtg gtc gag gaa atg atg tct cat tga gtattctcat gataacgcct         676
   Glu Val Val Glu Glu Met Met Ser His
           210                 215
   cgatgccgct ttagtaagtt atcataactg ccactggtcc atccacaaac gccactgaac     736
   gcaagctagc tacagacacg ctcatcacta tgacgtgtct gtatattaat aagctaaccc     796
   gcattgagtt aaccaataac ggattcc                                         823
<210> 4
   <211> 216
   <212> PRT
   <213> Escherichia coli
<400> 4
   Met Ala Ile Arg Leu Tyr Lys Leu Ala Val Ala Leu Gly Val Phe Ile
   1               5                   10                  15
   Val Ser Ala Pro Ala Phe Ser His Gly His His Ser His Gly Lys Pro
               20                  25                  30
   Leu Thr Glu Val Glu Gln Lys Ala Ala Asn Gly Val Phe Asp Asp Ala
           35                  40                  45
   Asn Val Gln Asn Arg Thr Leu Ser Asp Trp Asp Gly Val Trp Gln Ser
       50                  55                  60
   Val Tyr Pro Leu Leu Gln Ser Gly Lys Leu Asp Pro Val Phe Gln Lys
   65                  70                  75                  80
   Lys Ala Asp Ala Asp Lys Thr Lys Thr Phe Ala Glu Ile Lys Asp Tyr
                   85                  90                  95
   Tyr His Lys Gly Tyr Ala Thr Asp Ile Glu Met Ile Gly Ile Glu Asp
               100                 105                 110
   Gly Ile Val Glu Phe His Arg Asn Asn Glu Thr Thr Ser Cys Lys Tyr
           115                 120                 125
   Asp Tyr Asp Gly Tyr Lys Ile Leu Thr Tyr Lys Ser Gly Lys Lys Gly
       130                 135                 140
   Val Arg Tyr Leu Phe Glu Cys Lys Asp Pro Glu Ser Lys Ala Pro Lys
   145                 150                 155                 160
   Tyr Ile Gln Phe Ser Asp His Ile Ile Ala Pro Arg Lys Ser Ser His
                   165                 170                 175
   Phe His Ile Phe Met Gly Asn Asp Ser Gln Gln Ser Leu Leu Asn Glu
               180                 185                 190
   Met Glu Asn Trp Pro Thr Tyr Tyr Pro Tyr Gln Leu Ser Ser Glu Glu
           195                 200                 205
   Val Val Glu Glu Met Met Ser His
       210                 215
<210> 5
   <211> 651
   <212> DNA
   <213> Shigella flexneri
<220>
   <221> CDS
   <222> (1)..(651)
   <223> yodA coding region
<400> 5
   ttg gcg att cgt ctt cat aaa ctg gct gtt gct tta ggt gtc ttt att      48
   Met Ala Ile Arg Leu His Lys Leu Ala Val Ala Leu Gly Val Phe Ile
   1               5                   10                  15
   gtt agc gct cct gcc ttt tcg cat ggt cat cat tct cat ggc aaa ccc      96
   Val Ser Ala Pro Ala Phe Ser His Gly His His Ser His Gly Lys Pro
               20                  25                  30
   tta aca gag gtc gaa caa aaa gct gct aat ggt gtt ttt gat gat acc     144
   Leu Thr Glu Val Glu Gln Lys Ala Ala Asn Gly Val Phe Asp Asp Thr
           35                  40                  45
   aat gta caa aac cga acg ctc agt gac tgg gat gga gtc tgg caa tcc     192
   Asn Val Gln Asn Arg Thr Leu Ser Asp Trp Asp Gly Val Trp Gln Ser
       50                  55                  60
   gtt tac cct tta ctg caa agt ggc aaa ctt gat ccc gtc ttt cag aag     240
   Val Tyr Pro Leu Leu Gln Ser Gly Lys Leu Asp Pro Val Phe Gln Lys
   65                  70                  75                  80
   aaa gcg gat gca gat aaa acc aaa aca ttt gct gaa att aaa gat tat     288
   Lys Ala Asp Ala Asp Lys Thr Lys Thr Phe Ala Glu Ile Lys Asp Tyr
                   85                  90                  95
   tat cgc aaa ggt tat ccg aca gat atc gaa atg att ggc att gag gac     336
   Tyr Arg Lys Gly Tyr Pro Thr Asp Ile Glu Met Ile Gly Ile Glu Asp
               100                 105                 110
   ggc att gtc gaa ttc cat aga aat aat gaa aca aca tcc tgt aaa tat     384
   Gly Ile Val Glu Phe His Arg Asn Asn Glu Thr Thr Ser Cys Lys Tyr
           115                 120                 125
   gat tac gat gga tac aaa ata ctc acc tat aaa tca ggc aag aaa ggc     432
   Asp Tyr Asp Gly Tyr Lys Ile Leu Thr Tyr Lys Ser Gly Lys Lys Gly
       130                 135                 140
   gtt cgt tac tta ttc gaa tgt aaa gat cct gaa agc aaa gcc cct aaa     480
   Val Arg Tyr Leu Phe Glu Cys Lys Asp Pro Glu Ser Lys Ala Pro Lys
   145                 150                 155                 160
   tat ata caa ttt agc gat cat ata att gca cca cga aaa tca tct cat     528
   Tyr Ile Gln Phe Ser Asp His Ile Ile Ala Pro Arg Lys Ser Ser His
                   165                 170                 175
   ttt cac att ttt atg ggt aat gat tcc cag caa tct tta ttg aat gaa     576
   Phe His Ile Phe Met Gly Asn Asp Ser Gln Gln Ser Leu Leu Asn Glu
               180                 185                 190
   atg gaa aac tgg cca acg tat tat ccc tat cag ttg agt agc gaa gaa     624
   Met Glu Asn Trp Pro Thr Tyr Tyr Pro Tyr Gln Leu Ser Ser Glu Glu
           195                 200                 205
   gtg gtc gag gag atg atg tct cat tga                                 651
   Val Val Glu Glu Met Met Ser His
       210                 215
<210> 6
   <211> 216
   <212> PRT
   <213> Shigella flexneri
<400> 6
   Met Ala Ile Arg Leu His Lys Leu Ala Val Ala Leu Gly Val Phe Ile
   1               5                   10                  15
   Val Ser Ala Pro Ala Phe Ser His Gly His His Ser His Gly Lys Pro
               20                  25                  30
   Leu Thr Glu Val Glu Gln Lys Ala Ala Asn Gly Val Phe Asp Asp Thr
           35                  40                  45
   Asn Val Gln Asn Arg Thr Leu Ser Asp Trp Asp Gly Val Trp Gln Ser
       50                  55                  60
   Val Tyr Pro Leu Leu Gln Ser Gly Lys Leu Asp Pro Val Phe Gln Lys
   65                  70                  75                  80
   Lys Ala Asp Ala Asp Lys Thr Lys Thr Phe Ala Glu Ile Lys Asp Tyr
                   85                  90                  95
   Tyr Arg Lys Gly Tyr Pro Thr Asp Ile Glu Met Ile Gly Ile Glu Asp
               100                 105                 110
   Gly Ile Val Glu Phe His Arg Asn Asn Glu Thr Thr Ser Cys Lys Tyr
           115                 120                 125
   Asp Tyr Asp Gly Tyr Lys Ile Leu Thr Tyr Lys Ser Gly Lys Lys Gly
       130                 135                 140
   Val Arg Tyr Leu Phe Glu Cys Lys Asp Pro Glu Ser Lys Ala Pro Lys
   145                 150                 155                 160
   Tyr Ile Gln Phe Ser Asp His Ile Ile Ala Pro Arg Lys Ser Ser His
                   165                 170                 175
   Phe His Ile Phe Met Gly Asn Asp Ser Gln Gln Ser Leu Leu Asn Glu
               180                 185                 190
   Met Glu Asn Trp Pro Thr Tyr Tyr Pro Tyr Gln Leu Ser Ser Glu Glu
           195                 200                 205
   Val Val Glu Glu Met Met Ser His
       210                 215

## Description

### Field of the Invention

This invention relates to a process for preparing L-amino acids, in particular L-threonine, using recombinant microorganisms of the Enterobacteriaceae family in which the open reading frame (ORF) designated yodA is overexpressed, and to these microorganisms.

### Prior Art

L-Amino acids, in particular L-threonine, are used in human medicine and in the pharmaceutical industry, in the foodstuffs industry and, very particularly, in animal nutrition.

It is known that L-amino acids are prepared by fermenting strains of the Enterobacteriaceae, in particular Escherichia coli (E. coli) and Serratia marcescens. Because of the great importance, efforts are constantly being made to improve the preparation methods. Methodological improvements can relate to fermentation measures of a technical nature, such as stirring or supply with oxygen, or to the composition of the nutrient media, such as the sugar concentration during the fermentation, or to the working-up into product form, for example by means of ion exchange chromatography, or to the intrinsic performance characteristics of the microorganism itself.

Methods of mutagenesis, selection and mutant choice are used for improving the performance characteristics of these microorganisms. This makes it possible to obtain strains which are resistant to antimetabolites, such as the threonine analog α-amino-β-hydroxyvaleric acid (AHV), or auxotrophic for metabolites which are of regulatory importance, and produce L-amino acids such as L-threonine. For some years now, methods of recombinant DNA technology have also been used for improving L-amino acid-producing strains of the Enterobacteriaceae family, by amplifying individual amino acid biosynthesis genes and investigating the effect this has on production. Comprehensive information on the cell biology and molecular biology of Escherichia coli and Salmonella can be found in Neidhardt (ed): Escherichia coli and Salmonella, Cellular and Molecular Biology, 2nd edition, ASM Press, Washington, D.C., USA, (1996).

### Object of the Invention

The inventors have set themselves the object of providing novel measures for improving the fermentative preparation of L-amino acids, in particular L-threonine.

### Summary of the Invention

The invention relates to recombinant microorganisms of the Enterobacteriaceae family which contain an overexpressed yodA ORF, which encodes a polypeptide which is annotated as being a putative metal-binding protein, and which produce L-amino acids, in particular L-threonine, in an improved manner.

The microorganisms which are not recombinant for the yodA ORF, and which do not contain any potentiated yodA ORF, are in each case used as the starting point for the comparison.

These microorganisms include, in particular, microorganisms of the Enterobacteriaceae family in which a polynucleotide, which encodes a polypeptide whose amino acid sequence is at least 95%, preferably at least 98% or at least 99%, particularly preferably 99.5% and, very particularly preferably, 100%, identical to an amino acid sequence selected from the group SEQ ID No. 4 and SEQ ID No. 6, is potentiated.

Preference is given to amino acid sequences which are identical to those from SEQ ID No. 4 and SEQ ID No. 6. Said microorganisms contain overexpressed polynucleotides selected from the group:
a) polynucleotide having the nucleotide sequence SEQ ID No. 3 or SEQ ID No. 5;
b) polynucleotide having a nucleotide sequence which corresponds to SEQ ID No. 3 or SEQ ID No. 5 within the limits of the degeneracy of the genetic code;
c) polynucleotide sequence having a sequence which hybridizes, under stringent conditions, with the sequence which is complementary to the sequence SEQ ID No. 3 or SEQ ID No. 5;
d) polynucleotide having a sequence SEQ ID No. 3 or SEQ ID No. 5 which contains functionally neutral sense mutants,
with the polynucleotides encoding a putative metal-binding protein.

The invention also relates to a process for fermentatively preparing L-amino acids, in particular L-threonine, using recombinant microorganisms of the Enterobacteriaceae family which already produce L-amino acids and in which at least the open reading frame (ORF) designated yodA, or nucleotide sequences encoding its gene product, is/are overexpressed.

Preference is given to using the described microorganisms.

### Detailed Description of the Invention

The mention, in that which follows, of L-amino acids or amino acids means one or more amino acids, including their salts, selected from the group L-asparagine, L-threonine, L-serine, L-glutamate, L-glycine, L-alanine, L-cysteine, L-valine, L-methionine, L-proline, L-isoleucine, L-leucine, L-tyrosine, L-phenylalanine, L-histidine, L-lysine, L-tryptophan, L-arginine and L-homoserine. Particular preference is given to L-threonine.

In this connection, the term "potentiation" describes an increase in the intracellular activity or concentration of one or more enzymes or proteins, which are encoded by the corresponding DNA, in a microorganism, which increase is achieved, for example, by increasing the copy number of the gene or genes, or of the ORF or ORFs, by at least one (1) copy, by functionally linking a strong promoter to the gene, or by using a gene or allele or ORF which encodes a corresponding enzyme or protein having a high activity, or by, where appropriate, combining these measures.

A segment of a nucleotide sequence which encodes, or can encode, a protein or polypeptide or ribonucleic acid to which the prior art cannot assign any function is described as being an open reading frame (ORF). After a function has been assigned to the nucleotide sequence segment in question, the segment is generally referred to as being a gene. In general, alleles are understood as being alternative forms of a given gene. The forms are characterized by differences in their nucleotide sequences.

In general, the protein encoded by a nucleotide sequence, i.e. an ORF, a gene or an allele, or the encoded ribonucleic acid, is described as being a gene product.

In general, the measures of potentiation, in particular overexpression, increase the activity or concentration of the corresponding protein by at least 10%, 25%, 50%, 75%, 100%, 150%, 200%, 300%, 400% or 500%, at most up to 1000% or 2000%, based on that of the wild-type protein or on the activity or concentration of the protein in the microorganism or parent strain which is not recombinant for the corresponding enzyme or protein. A nonrecombinant microorganism or parent strain is understood as being the microorganism on which the measures according to the invention are implemented.

The invention relates to a process for preparing L-amino acids by fermenting recombinant microorganisms of the Enterobacteriaceae family, which process is characterized in that
a) the microorganisms as defined in claims 1-14 are cultured in a medium under conditions under which the desired L-amino acid is enriched in the medium or in the cells, and
b) the desired L-amino acid is isolated, with, where appropriate, constituents of the fermentation broth, and/or the biomass, in its entirety or in portions (≥ 0 to 100%), remaining in the isolated product or being completely removed.

The microorganisms, which are recombinant, which contain an overexpressed open reading frame (ORF) designated yodA, and which are likewise part of the subject-matter of the present invention, can produce L-amino acids from glucose, sucrose, lactose, fructose, maltose, molasses, where appropriate starch and where appropriate cellulose, or from glycerol and ethanol. The microorganisms are representatives of the Enterobacteriaceae family, selected from the genera Escherichia, Erwinia, Providencia and Serratia. The genera Escherichia and Serratia are preferred. The species Escherichia coli and Serratia marcescens are to be mentioned, in particular, in the case of the genus Escherichia and, respectively, in the case of the genus Serratia.

In general, recombinant microorganisms are produced by transformation, transduction or conjugation, or a combination of these methods, with a vector which contains the desired ORF, the desired gene, an allele of this ORF or gene, or parts thereof, and/or a promoter which potentiates the expression of the ORF or gene. This promoter can be the promoter which has been produced, as a result of potentiating mutation, from the endogenous regulatory sequence located upstream of the gene or ORF, or an efficient promoter can have been fused to the gene or ORF.

Examples of suitable, in particular L-threonine-producing, strains of the genus Escherichia, in particular of the species Escherichia coli, are

| | |
|---|---|
| - Escherichia coli H4581 | (EP 0 301 572) |
| - Escherichia coli KY10935 | (Bioscience Biotechnology and Biochemistry 61(11):1877-1882 (1997) |
| - Escherichia coli VNIIgenetika MG442 | (US-A-4278 765) |
| - Escherichia coli VNIIgenetika M1 | (US-A-4,321,325) |
| - Escherichia coli VNIIgenetika 472T23 | (US-A-5,631,157) |
| - Escherichia coli BKIIM B-3996 | (US-A-5,175,107) |
| - Escherichia coli kat 13 | (WO 98/04715) |
| - Escherichia coli KCCM-10132 | (WO 00/09660) |

Examples of suitable L-threonine-producing strains of the genus Serratia, in particular of the species Serratia marcescens, are
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-Threonine-producing strains from the Enterobacteriaceae family preferably possess, inter alia, one or more of the genetic or phenotypic features selected from the group:
resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidine, resistance to cyclopentanecarboxylic acid, resistance to rifampicin, resistance to valine analogs such as valine hydroxamate, resistance to purine analogs, such as 6-dimethylaminopurine, requirement for L-methionine, where appropriate partial and compensatable requirement for L-isoleucine, requirement for meso-diaminopimelic acid, auxotrophy with regard to threonine-containing dipeptides, resistance to L-threonine, resistance to threonine raffinate, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity towards fluoropyruvate, defective threonine dehydrogenase, where appropriate ability to utilize sucrose, potentiation of the threonine operon, potentiation of homoserine dehydrogenase I-aspartate kinase I, preferably of the feedback-resistant form, potentiation of homoserine kinase, potentiation of threonine synthase, potentiation of aspartate kinase, where appropriate of the feedback-resistant form, potentiation of aspartate semialdehyde dehydrogenase, potentiation of phosphoenolpyruvate carboxylase, where appropriate of the feedback-resistant form, potentiation of phosphoenolpyruvate synthase, potentiation of transhydrogenase, potentiation of the RhtB gene product, potentiation of the RhtC gene product, potentiation of the YfiK gene product, potentiation of a pyruvate carboxylase and attenuation of acetic acid formation.

It has been found that, after the yodA gene or open reading frame (ORF), or its alleles, has/have been overexpressed, microorganisms of the Enterobacteriaceae family produce L-amino acids, in particular L-threonine, in an improved manner.

The nucleotide sequences of the genes or open reading frames (ORFs) of Escherichia coli belong to the prior art and can be obtained from the Escherichia coli genome sequence published by Blattner et al. (Science 277: 1453-1462 (1997)). It is known that enzymes which are endogenous to the host (methionine aminopeptidase) can cleave off the N-terminal amino acid methionine.

The nucleotide sequences for the yodA ORF are likewise known from Shigella flexneri, which also belongs to the Enterobacteriaceae family.

The Escherichia coli K12 yodA ORF is described, inter alia, by the following particulars:

| | |
|---|---|
| Designation: | open reading frame |
| Function: | annotated as being a putative metal-binding protein; expression increased in connection with cadmium and hydrogen peroxide stress |
| Description: | the yodA open reading frame encodes a 24.8 KD protein; the isoelectric point is 5.9; being chromosomally located, it is present, for example in the case of Escherichia coli K12 MG1655, in the intergenic region between the open reading frame b1972, encoding a putative membrane protein, and the yodB ORF, encoding a putative cytochrome b |
| References: | Blattner et al., Science 5; 277 (5331): 1453-1474 (1997) |
| | Puskarova et al., Microbiology 148 (PT 12): 3801-3811 (2002), |
| | David et al., Acta Crystallographica Section D. Biological Crystallography 58(PT 7): 1243-1245 (2002), |
| | David et al., The Journal of Biological Chemistry 278(44): 43728-43735 (2003) |
| Accession No.: | AE000288 |
| Alternative gene name: | b1973 |

The nucleic acid sequences can be obtained from the databases belonging to the National Center for Biotechnology Information (NCBI) of the National Library of Medicine (Bethesda, MD, USA), from the nucleotide sequence database belonging to the European Molecular Biologies Laboratories (EMBL, Heidelberg, Germany and Cambridge, UK) or from the Japanese DNA database (DDBJ, Mishima, Japan).

For the sake of greater clarity, the known nucleotide sequence of the Escherichia coli yodA ORF is given in SEQ ID No. 3 and the known sequence for the Shigella flexneri (AE015219) yaaU-ORF is given in SEQ ID No. 5. The amino acid sequences of the proteins encoded by these reading frames are depicted as SEQ ID No. 4 and, respectively, SEQ ID No. 6.

The open reading frames described in the given text passages can be used in accordance with the invention. It is furthermore possible to use alleles of the genes or open reading frames which ensue from the degeneracy of the genetic code or as a result of functionally neutral sense mutations. The use of endogenous genes or endogenous open reading frames is preferred.

"Endogenous genes" or "endogenous nucleotide sequences" are understood as being the genes or open reading frames, or alleles or nucleotide sequences which are present in the population of a species.

Disclosed is that the suitable alleles of the yodA ORF which contain functionally neutral sense mutations include, inter alia, those which lead to at most 20 or to at most 10, preferably to at most 5 or to at most 3, very particularly preferably to at most 2 or to at most one, conservative amino acid substitution in the protein which they encode.

In the case of the aromatic amino acids, the substitutions are said to be conservative when phenylalanine, tryptophan and tyrosine are substituted for each other. In the case of the hydrophobic amino acids, the substitutions are said to be conservative when leucine, isoleucine and valine are substituted for each other. In the case of the polar amino acids, the substitutions are said to be conservative when glutamine and asparagine are substituted for each other. In the case of the basic amino acids, the substitutions are said to be conservative when arginine, lysine and histidine are substituted for each other. In the case of the acidic amino acids, the substitutions are said to be conservative when aspartic acid and glutamic acid are substituted for each other. In the case of the hydroxyl group-containing amino acids, the substitutions are said to be conservative when serine and threonine are substituted for each other.

Also disclosed is that in the same way, it is also possible to use those nucleotide sequences which encode variants of said proteins which additionally contain, as the N terminus or C terminus, an extension or truncation by at least one (1) amino acid. This extension or truncation amounts to not more than 50, 40, 30, 20, 10, 5, 3 or 2 amino acids or amino acid residues.

The suitable alleles also include those which encode proteins in which at least one (1) amino acid has been inserted or deleted. The maximum number of such changes, designated indels, can affect 2, 3, 5, 10 or 20, in no case, however, more than 30, amino acids.

The suitable alleles furthermore include those which can be obtained by hybridization, in particular under stringent conditions, using SEQ ID No. 3 or SEQ ID No. 5, or parts thereof, in particular the coding regions, or the sequences which are complementary thereto.

The skilled person can find instructions for identifying DNA sequences by means of hybridization in, inter alia, the manual "The DIG System Users Guide for Filter Hybridization" from the company Boehringer Mannheim GmbH (Mannheim, Germany, 1993) and in Liebl et al. (International Journal of Systematic Bacteriology 41: 255-260 (1991)). The hybridization takes place under stringent conditions; that is, the only hybrids to be formed are those in which probe and target sequence, i.e. the polynucleotides treated with the probe, are at least 80% identical. It is known that the stringency of the hybridization, including the washing steps, is influenced, or determined, by varying the buffer composition, the temperature and the salt concentration. The hybridization reaction is generally carried out at a stringency which is relatively low as compared with that of the washing steps (Hybaid Hybridisation Guide, Hybaid Limited, Teddington, UK, 1996).

For example, a buffer corresponding to 5x SSC buffer can be used, at a temperature of approx. 50°C-68°C, for the hybridization reaction. Under these conditions, probes can also hybridize with polynucleotides which exhibit less than 80% identity with the sequence of the probe. These latter hybrids are less stable and are removed by washing under stringent conditions. This can be achieved, for example, by lowering the salt concentration down to 2x SSC and, where appropriate, subsequently to 0.5× SSC (The DIG System User's Guide for Filter Hybridisation, Boehringer Mannheim, Mannheim, Germany, 1995), with the temperature being adjusted to approx. 50°C-68°C, approx. 52°C-68°C, approx. 54°C-68°C, approx. 56°C-68°C, approx. 58°C-68°C, approx. 60°C-68°C, approx. 62°C-68°C, approx. 64°C-68°C, or approx. 66°C-68°C. Temperature ranges of approx. 64°C-68°C or approx. 66°C-68°C are preferred. It is possible, where appropriate, to lower the salt concentration down to a concentration corresponding to 0.2× SSC or 0.1× SSC. Polynucleotide fragments which possess, for example, at least 80% or at least 90%, at least 91%, at least 92%, at least 93%, at least 94%, at least 95%, at least 96%, at least 97%, at least 98%, or at least 99%, identity with the sequence of the probe which is employed, or with the nucleotide sequences depicted in SEQ ID No. 3 or SEQ ID No. 5, can be isolated by increasing the hybridization temperature stepwise from 50°C to 68°C in steps of approx. 1-2°C. Further instructions for the hybridization can be obtained commercially in the form of kits (e.g. DIG Easy Hyb from the company Roche Diagnostics GmbH, Mannheim, Germany, Catalog No. 16035558). The nucleotide sequences which are thus obtained encode polypeptides which possess at least 90%, in particular at least 95%, preferably at least 98% or at least 99%, very particularly preferably 99.5%, identity with the amino acid sequences depicted in SEQ ID No. 4 or SEQ ID No. 6.

In order to achieve a potentiation, it is possible, for example, to increase the expression of the genes or open reading frames or alleles. Also disclosed is to increase the catalytic properties of the protein. Both measures can be combined, where appropriate.

In order to achieve overexpression, it is possible, for example, to increase the copy number of the corresponding genes or open reading frames or to mutate the promoter and regulatory region or the ribosomal binding site which is located upstream of the structural gene. Expression cassettes which are incorporated upstream of the structural gene act in the same way. By incorporating inducible promoters, it is additionally possible to increase expression during the course of the fermentative L-amino acid, in particular L-threonine, production; it can furthermore also be advantageous to use, for the gene expression, promoters which enables a different chronological gene expression to be obtained. The expression is likewise improved by measures for extending the lifetime of the m-RNA. The enzyme activity is furthermore also potentiated by preventing the enzyme protein from being broken down. The ORFs, genes or gene constructs can either be present in plasmids having different copy numbers or be integrated, and amplified, in the chromosome. As an alternative, it is also possible to achieve overexpression of the genes concerned by altering the composition of the medium and the conduct of the culture.

Methods for achieving overexpression are adequately described in the prior art, for example in Makrides et al. (Microbiological Reviews 60 (3), 512-538 (1996)). Using vectors increases the copy number by at least one (1) copy. The vectors used can be plasmids, as described, for example, in US 5,538,873. The vectors used can likewise be phages, for example the phage Mu, as described in EP 0 332 448, or the phage lambda (λ). An increase in the copy number can also be achieved by incorporating an additional copy into another site in the chromosome, for example into the phage λ att site (Yu and Court, Gene 223, 77-81 (1998)). US 5,939,307 reports that it was possible to increase expression by incorporating expression cassettes or promoters, such as the tac promoter, the trp promoter, the lpp promoter, or the phage λ P_{L} promoter or P_{R} promoter, for example upstream of the chromosomal threonine operon. It is possible to use the T7 phage promoters, the gearbox promoters or the nar promoter in the same way. Expression cassettes or promoters of this nature can also be used to ovrerexpress plasmid-bound genes, as described in EP 0 593 792. Using the lacI^{Q} allele makes it possible, in turn, to control the expression of plasmid-bound genes (Glascock and Weickert, Gene 223, 221-231 (1998)). It is furthermore possible for the activities of the promoters to be increased by modifying their sequences by means of using insertion(s) and/or deletion(s) to effect one or more nucleotide substitutions. A different chronological gene expression can be achieved, for example, by using the growth phase-dependent fis promoter, as described in Walker et al. (Journal of Bacteriology 181: 1269-80 (1999)).

The skilled person can find general instructions in this regard in, inter alia, Chang and Cohen (Journal of Bacteriology 134: 1141-1156 (1978)), Hartley and Gregori (Gene 13: 347-353 (1981)), Amann and Brosius (Gene 40: 183-190 (1985)), de Broer et al. (Proceedings of the National Academy of Sciences of the United States of America 80: 21-25 (1983)), LaVallie et al. (BIO/TECHNOLOGY 11: 187-193 (1993)), PCT/US97/13359, Llosa et al. (Plasmid 26: 222-224 (1991)), Quandt and Klipp (Gene 80: 161-169 (1989)), Hamilton et al. (Journal of Bacteriology 171: 4617-4622 (1989)), and Jensen and Hammer (Biotechnology and Bioengineering 58: 191-195 (1998)), and in known text books dealing with genetics and molecular biology.

It is possible to use plasmid vectors which can be replicated in Enterobacteriaceae, such as pACYC184-derived cloning vectors (Bartolomé et al.; Gene 102: 75-78 (1991)), pTrc99A (Amann et al.; Gene 69: 301-315 (1988)) or pSC101 derivatives (Vocke and Bastia; Proceedings of the National Academy of Sciences USA 80(21): 6557-6561 (1983)). In one process according to the invention, it is possible to use a plasmid vector-transformed strain, with the plasmid vector carrying at least one nucleotide sequence or allele encoding the yodA ORF or its gene product.

The term "transformation" is understood as meaning the uptake of an isolated nucleic acid by a host (microorganism).

It is likewise possible to use sequence exchange (Hamilton et al.; Journal of Bacteriology 171: 4617-4622 (1989)), conjugation or transduction to transfer mutations, which affect the expression of the respective genes or open reading frames, into different strains.

More detailed explanations with regard to the concepts of genetics and molecular biology can be found in known text books dealing with genetics and molecular biology, such as the text book by Birge (Bacterial and Bacteriophage Genetics, 4th ed., Springer Verlag, New York (USA), 2000) or the text book by Berg, Tymoczko and Stryer (Biochemistry, 5th ed., Freeman and Company, New York (USA), 2002) or the manual of Sambrook et al. (Molecular Cloning, A Laboratory Manual, (3-Volume Set), Cold Spring Harbor Laboratory Press, Cold Spring Harbor (USA), 2001).

When using strains of the Enterobacteriaceae family to produce L-amino acids, in particular L-threonine, it can also be advantageous, in addition to overexpressing the yodA open reading frame, to potentiate one or more enzymes of the known threonine biosynthesis pathway, or enzymes of anaplerotic metabolism, or enzymes for producing reduced nicotinamide adenine dinucleotide phosphate, or enzymes of glycolysis, or PTS enzymes, or enzymes of sulfur metabolism. Preference is generally given to using endogenous genes.

Thus, it is possible, for example, to simultaneously potentiate, in particular overexpress, one or more of the genes selected from the group
- at least one gene of the thrABC operon encoding aspartate kinase, homoserine dehydrogenase, homoserine kinase and threonine synthase (US-A-4,278,765),
- the Corynebacterium glutamicum pyruvate carboxylase-encoding pyc gene (WO 99/18228),
- the phosphoenolpyruvate synthase-encoding pps gene (Molecular and General Genetics 231(2): 332-336 (1992)),
- the phosphoenolpyruvate carboxylase-encoding ppc gene (WO 02/064808),
- the pntA and pntB genes encoding the pyridine transhydrogenase subunits (European Journal of Biochemistry 158: 647-653 (1986)),
- the rhtB gene encoding the homoserine resistance-mediating protein (EP-A-0 994 190),
- the rhtC gene encoding the threonine resistance-mediating protein (EP-A-1 013 765),
- the Corynebacterium glutamicum thrE gene encoding the threonine export carrier protein (WO 01/92545),
- the glutamate dehydrogenase-encoding gdhA gene (Nucleic Acids Research 11: 5257-5266 (1983); Gene 23: 199-209 (1983)),
- the phosphoglucomutase-encoding pgm gene (WO 03/004598),
- the fructose biphosphate aldolase-encoding fba gene (WO 03/004664),
- the ptsHIcrr operon ptsH gene encoding the phosphohistidine protein hexose phosphotransferase of the phosphotransferase system PTS (WO 03/004674),
- the ptsHIcrr operon ptsI gene encoding the enzyme I of the phosphotransferase system PTS (WO 03/004674),
- the ptsHIcrr operon crr gene encoding the glucose-specific IIA component of the phosphotransferase system PTS (WO 03/004674),
- the ptsG gene encoding the glucose-specific IIBC component (WO 03/004670),
- the lrp gene encoding the leucine regulon regulator (WO 03/004665),
- the fadR gene encoding the fad regulon regulator (WO 03/038106),
- the iclR gene encoding the central intermediary metabolism regulator (WO 03/038106),
- the ahpCF operon ahpC gene encoding the small subunit of alkyl hydroperoxide reductase (WO 03/004663),
- the ahpCF operon ahpF gene encoding the large subunit of alkyl hydroperoxide reductase (WO 03/004663),
- the cysteine synthase A-encoding cysK gene (WO 03/006666),
- the cysB gene encoding the cys regulon regulator (WO 03/006666),
- the cysJIH operon cysJ gene encoding the NADPH sulfite reductase flavoprotein (WO 03/006666),
- the cysJIH operon cysI gene encoding the NADPH sulfite reductase hemoprotein (WO 03/006666),
- the cysJIH operon cysH gene encoding adenylyl sulfate reductase (WO 03/006666),
- the rseABC operon rseA gene encoding a membrane protein possessing anti-sigmaE activity (WO 03/008612),
- the rseABC operon rseC gene encoding a global regulator of the SigmaE factor (WO 03/008612),
- the sucABCD operon sucA gene encoding the decarboxylase subunit of 2-ketoglutarate dehydrogenase (WO 03/008614),
- the sucABCD operon sucB gene encoding the dihydrolipoyl transsuccinase E2 subunit of 2-ketoglutarate dehydrogenase (WO 03/008614),
- the suc ABCD operon sucC gene encoding the β subunit of succinyl-CoA synthetase (WO 03/008615),
- the sucABCD operon sucD gene encoding the α subunit of succinyl-CoA synthetase (WO 03/008615),
- the aceE gene encoding the E1 component of the pyruvate dehydrogenase complex (WO 03/076635),
- the aceF gene encoding the E2 component of the pyruvate dehydrogenase complex (WO 03/076635),
- the rseB gene encoding the SigmaE factor activity regulator (Molecular Microbiology 24(2): 355-371 (1997)),
- the gene product of the Escherichia coli yaaU open reading frame (ORF) (Accession Number AE005181 of the National Center for Biotechnology Information (NCBI, Bethesda, MD, USA, (DE10361268.8)).

For the purpose of producing L-amino acids, in particular L-threonine, it can also be advantageous, in addition to overexpressing the yodA open reading frame, to attenuate, in particular eliminate or reduce, the expression of one or more of the genes selected from the group
- the threonine dehydrogenase-encoding tdh gene (Journal of Bacteriology 169: 4716-4721 (1987)),
- the malate dehydrogenase (E.C. 1.1.1.37)-encoding mdh gene (Archives in Microbiology 149: 36-42 (1987)),
- the gene product of the Escherichia coli yjfA open reading frame (ORF) (Accession Number AAC77180 of the National Center for Biotechnology Information (NCBI, Bethesda, MD, USA, (WO 02/29080)),
- the gene product of the Escherichia coli ytfP open reading frame (ORF) (Accession Number AAC77179 of the National Center for Biotechnology Information (NCBI, Bethesda, MD, USA, (WO 02/29080)),
- the pckA gene encoding the enzyme phosphoenolpyruvate carboxykinase (WO 02/29080),
- the pyruvate oxidase-encoding poxB gene (WO 02/36797),
- the dgsA gene (WO 02/081721), which is also known under the designation mlc gene, encoding the DgsA regulator of the phosphotransferase system,
- the fructose repressor-encoding fruR gene (WO 02/081698), which is also known under the designation cra gene,
- the Sigma³⁸ factor-encoding rpoS gene (WO 01/05939), which is also known under the designation katF gene, and
- the aspartate ammonium lyase-encoding aspA gene (WO 03/008603).

In this connection, the term "attenuation" describes the reduction or elimination of the intracellular activity or concentration of one or more enzymes or proteins, which are encoded by the corresponding DNA, in a microorganism, with, for example, use being made of a promoter which is weaker than in the microorganism or parent strain which is not recombinant for the corresponding enzyme or protein, or of a gene or allele which encodes a corresponding enzyme or protein having a lower activity, or the corresponding enzyme or protein, or the open reading frame or the gene, being inactivated, and, where appropriate, these measures being combined.

The measures of attenuation in general lower the activity or concentration of the corresponding protein down to from 0 to 75%, from 0 to 50%, from 0 to 25%, from 0 to 10%, or from 0 to 5%, of the activity or concentration of the wild-type protein or of the activity or concentration of the protein in the microorganism or parent strain which is for the corresponding enzyme or protein. The nonrecombinant microorganism or parent strain is understood as being the microorganism on which the measures according to the invention are implemented.

In order to achieve an attenuation, it is possible, for example, for the expression of the genes or open reading frames, or the catalytic properties of the enzyme proteins, to be reduced or eliminated. Both measures can be combined, where appropriate.

Gene expression can be reduced by means of conducting the culture in a suitable manner, by means of genetically altering (mutating) the signal structures for gene expression or else by means of antisense RNA technology. Examples of signal structures for gene expression are repressor genes, activator genes, operators, promoters, attenuators, ribosome binding sites, the start codon and terminators. The skilled person can, for example, find information in this regard in, inter alia, Jensen and Hammer (Biotechnology and Bioengineering 58: 191-195 (1998)), Carrier and Keasling (Biotechnology Progress 15: 58-64 (1999)), Franch and Gerdes (Current Opinion in Microbiology 3: 159-164 (2000)) and known text books dealing with genetics and molecular biology, such as the text book by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art. Examples which may be mentioned are the studies by Qiu and Goodman (Journal of Biological Chemistry 272: 8611-8617 (1997)), Yano et al. (Proceedings of the National Academy of Sciences of the United States of America 95: 5511-5515 (1998)), Wente and Schachmann (Journal of Biological Chemistry 266: 20833-20839 (1991)). Comprehensive accounts can be obtained from known text books dealing with genetics and molecular biology, such as that by Hagemann ("Allgemeine Genetik [General genetics]", Gustav Fischer Verlag, Stuttgart, 1986).

Mutations which come into consideration are transitions, transversions, insertions and deletions of at least one (1) base pair or nucleotide. Depending on the effect which the amino acid substitution produced by the mutation has on the enzyme activity, reference is made to missense mutations or to nonsense mutations. The missense mutation leads to the substitution of a given amino acid in a protein with another amino acid, with the amino acid substitution being, in particular, a nonconservative amino acid substitution. This substitution impairs the functional ability or activity of the protein and reduces it down to a value of from 0 to 75%, from 0 to 50%, from 0 to 25%, from 0 to 10% or from 0 to 5%. The nonsense mutation leads to a stop codon being formed in the coding region of the gene and, as a result, to translation being terminated prematurely. Insertions or deletions of at least one base pair in a gene lead to frame shift mutations, which in turn result in incorrect amino acids being incorporated or in the translation being terminated prematurely. If the mutation results in a stop codon being formed in the coding region, this then likewise leads to translation being terminated prematurely. Deletions of at least one (1) or more codons typically also lead to complete loss of the enzyme activity.

Instructions for generating these mutations belong to the prior art and can be obtained from known text books dealing with genetics and molecular biology, such as the text book by Knippers ("Molekulare Genetik [Molecular genetics]", 6th edition, Georg Thieme Verlag, Stuttgart, Germany, 1995), that by Winnacker ("Gene und Klone [Genes and clones]", VCH Verlagsgesellschaft, Weinheim, Germany, 1990) or that by Hagemann ("Allgemeine Genetik [General genetics]", Gustav Fischer Verlag, Stuttgart, 1986).

Suitable mutations in the genes can be incorporated into suitable strains by means of gene or allele substitution.

A common method is the method, described by Hamilton et al. (Journal of Bacteriology 171: 4617-4622 (1989)), of gene substitution using a conditionally replicating pSC101 derivate, pMAK705. Other methods described in the prior art, such as that of Martinez-Morales et al. (Journal of Bacteriology 181: 7143 -7148 (1999)) or that of Boyd et al. (Journal of Bacteriology 182: 842-847 (2000)), can also be used.

It is likewise possible to transfer mutations in the respective genes, or mutations which affect the expression of the respective genes or open reading frames, into different strains by means of conjugation or transduction.

For the purpose of producing L-amino acids, in particular L-threonine, it can also be advantageous, in addition to overexpressing the yodA open reading frame, to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms which have been prepared in accordance with the invention can be cultured in a batch process, in a fed-batch process, in a repeated fed-batch process or in a continuous process (DE102004028859.3 or US 5,763,230). A summary of known culture methods is given in the text book by Chmiel (Bioprozesstechnik 1. Einführung in die Bioverfahrenstechnik [Bioprocess technology 1. Introduction to bioprocess technology] (Gustav Fischer Verlag, Stuttgart, 1991)) or in the text book by Storhas (Bioreaktoren und periphere Einrichtungen [Bioreactors and peripheral installations] (Vieweg Verlag, Braunschweig/ Wiesbaden, 1994)).

The culture medium which is to be used must adequately satisfy the requirements of the given strains. The manual "Manual of Methods for General Bacteriology" from the American Society for Bacteriology (Washington D.C., USA, 1981) contains descriptions of culture media for different microorganisms.

Sugars and carbohydrates, such as glucose, sucrose, lactose, fructose, maltose, molasses, starch and, where appropriate, cellulose, oils and fats, such as soybean oil, sunflower oil, peanut oil and coconut fat, fatty acids, such as palmitic acid, stearic acid and linoleic acid, alcohols, such as glycerol and ethanol, and organic acids, such as acetic acid, can be used as the carbon source. These substances can be used individually or as a mixture.

Organic nitrogen-containing compounds, such as peptones, yeast extract, meat extract, malt extract, cornsteep liquor, soybean flour and urea, or inorganic compounds, such as ammonium sulfate, ammonium chloride, ammonium phosphate, ammonium carbonate and ammonium nitrate, can be used as the nitrogen source. The nitrogen sources can be used individually or as mixtures.

Phosphoric acid, potassium dihydrogen phosphate or dipotassium hydrogen phosphate, or the corresponding sodium-containing salts, can be used as the phosphorus source. In addition, the culture medium has to contain metal salts, such as magnesium sulfate or iron sulfate, which are required for growth. Finally, it is possible to use essential growth-promoting substances, such as amino acids and vitamins, in addition to the above-mentioned substances. Moreover, suitable precursors can be added to the culture medium. The above-mentioned addition substances can be added to the culture in the form of a once-only mixture or else fed in, in a suitable manner, as the culture proceeds.

In general, the fermentation is carried out at a pH of from 5.5 to 9.0, in particular from 6.0 to 8.0. Basic compounds, such as sodium hydroxide, potassium hydroxide, ammonia or ammonia water, or acidic compounds, such as phosphoric acid or sulfuric acid, are used, in a suitable manner, for controlling the pH of the culture. Antifoamants, such as fatty acid polyglycol esters, can be used for controlling foaming. Suitable substances having a selective action, for example antibiotics, can be added to the medium in order to maintain the stability of plasmids. Oxygen or oxygen-containing gas mixtures, such as air, is/are introduced into the culture in order to maintain aerobic conditions. The temperature of the culture is normally from 25°C to 45°C and preferably from 30°C to 40°C. The culturing is continued until a maximum of L-amino acids or L-threonine has been formed. This objective is normally achieved within 10 to 160 hours.

L-Amino acids can be analyzed by means of anion exchange chromatography followed by ninhydrin derivatization, as described in Spackman et al. (Analytical Chemistry 30: 1190-1206 (1958)); otherwise, they can be analyzed by reversed phase HPLC as described in Lindroth et al. (Analytical Chemistry 51: 1167-1174 (1979)).

The process according to the invention is used for fermentatively preparing L-amino acids, such as L-threonine, L-isoleucine, L-valine, L-methionine, L-homoserine, L-tryptophan and L-lysine, in particular L-threonine.

The following microorganism has been deposited in the Deutsche Sammlung für Mikroorganismen und Zellkulturen [German collection of microorganisms and cell cultures] (DSMZ, Braunschweig, Germany) in accordance with the Budapest Treaty:
- Escherichia coli strain E. coli MG442, as DSM 16574.

The present invention is clarified below with the aid of implementation examples.

The minimal (M9) and complete (LB) media for Escherichia coli which are used are described by J.H. Miller (A short course in bacterial genetics (1992), Cold Spring Harbor Laboratory Press). The isolation of plasmid DNA from Escherichia coli, and all the techniques for restriction, ligation and treatment with Klenow phosphatase and alkali phosphatase, are carried out in accordance with Sambrook et al. (Molecular Cloning - A Laboratory Manual (1989) Cold Spring Harbor Laboratory Press). Unless otherwise described, Escherichia coli is transformed in accordance with Chung et al. (Proceedings of the National Academy of Sciences of the United States of America 86: 2172-2175 (1989)).

The incubation temperature when preparing strains and transformants is 37°C.

### Example 1

### Constructing the expression plasmid pTrc99AyodA

The E. coli K12 yodA open reading frame is amplified using the polymerase chain reaction (PCR) and synthetic oligonucleotides. PCR primers are synthesized (MWG Biotech, Ebersberg, Germany) on the basis of the nucleotide sequence of the yodA open reading frame in E. coli K12 MG1655 (Accession Number AE000288, Blattner et al. (Science 277: 1453-1474 (1997)). The primers contain sequences for restriction enzymes, with these sequences being marked by underlining in the nucleotide sequences which are depicted below. The primer yodA_5' contains the restriction cleavage site for NcoI, while the primer yodA_3' contains that for HincII.
yodA_5' 5'- GGTCCATGGCGATTCGTCTTTACAAACTGG-3' (SEQ ID No. 1)
yodA_3' 5'- GGAATCCGTTATTGGTTAACTC - 3' (SEQ ID No. 2)

The chromosomal E. coli K12 MG1655 DNA which is employed for the PCR is isolated, in accordance with the manufacturer's instructions, using "Qiagen Genomic-tips 100/G" (QIAGEN, Hilden, Germany). The specific primers are able to amplify a DNA fragment of approx. 830 bp in size (SEQ ID No. 3) under standard PCR conditions (Innis et al. (1990) PCR Protocols. A Guide to Methods and Applications, Academic Press) using Pfu DNA polymerase (Promega Corporation, Madison, USA).

The PCR product is ligated, in accordance with the manufacturer's instructions, to the vector pCR-Blunt II-TOPO (Zero Blunt TOPO cloning kit, Invitrogen, Groningen, the Netherlands), in accordance with the manufacturer's instructions, and the latter is transformed into the E. coli strain TOP10. Plasmid-harboring cells are selected from LB agar to which kanamycin has been added to a concentration of 50 µg/ml. After the plasmid DNA has been isolated, the vector is cleaved with the restriction enzymes EcoRI and EcoRV and, after having been checked by being separated in an 0.8% agarose gel, is designated pCRBluntyodA.

The vector pCRBluntyodA is then restricted with the restriction enzymes NcoI and HincII and the yodA fragment is isolated using a QIAquick gel extraction kit (QIAGEN, Hilden, Germany) after it has been separated in an 0.8% agarose gel. The vector pTrc99A (Pharmacia Biotech, Uppsala, Sweden) is cleaved with the enzymes NcoI and SmaI and ligated to the isolated yodA fragment. The E. coli strain XL1-Blue MRF' (Stratagene, La Jolla, USA) is transformed with the ligation mixture and plasmid-harboring cells are selected on LB agar to which ampicillin has been added to a concentration of 50 µg/ml. After the plasmid DNA had been isolated, the fact that the cloning has been successful can be demonstrated by carrying out a control cleavage with the enzyme EcoRV.

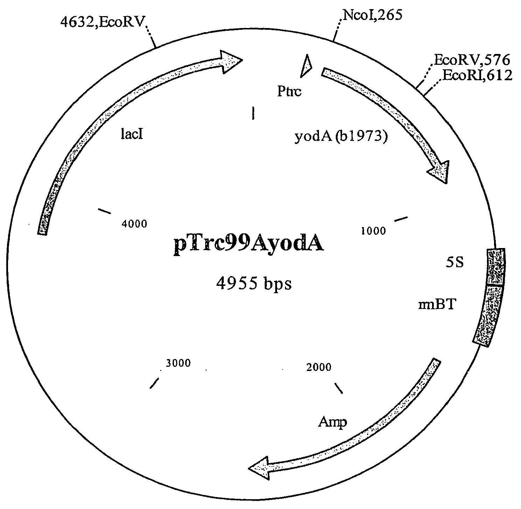

The plasmid is designated pTrc99AyodA (Figure 1).

### Example 2

### Preparing L-threonine using the strain MG442/pTrc99AyodA

The L-threonine-producing E. coli strain MG442 is described in the patent specification US-A-4,278,765 and has been deposited in the Russian national collection of industrial microorganisms (VKPM, Moscow, Russia) as CMIM B-1628 and in the Deutsche Sammlung fur Mikroorganisms und Zellkulturen [German collection of microorganisms and cell cultures] (DSMZ, Braunschweig, Germany), in accordance with the Budapest Treaty, as DSM 16574.

The strain MG442 is transformed with the expression plasmid pTrc99AyodA, as described in example 1, and with the vector pTrc99A, and plasmid-harboring cells are selected on LB agar containing 50 µg of ampicillin/ml. This results in the strains MG442/pTrc99AyodA and MG442/pTrc99A. Selected individual colonies are then propagated further on minimal medium having the following composition: 3.5 g of Na₂HPO₄*2H₂O/l, 1.5 g of KH₂PO₄/l, 1 g of NH₄Cl/l, 0.1 g of MgSO₄*7H₂O/l, 2 g of glucose/l, 20 g of agar/l and 50 mg of ampicillin/l.

The formation of L-threonine is examined in 10 ml batch cultures which are contained in 100 ml Erlenmeier flasks. For this, 10 ml of preliminary culture medium of the following composition: 2 g of yeast extract/l, 10 g of (NH₄)₂SO₄/l, 1 g of KH₂PO₄/l, 0.5 g of MgSO₄*7H₂O/l, 15 g of CaCO₃/l, 20 g of glucose/l and 50 mg of ampicillin/l are inoculated and incubated, at 37°C and 180 rpm for 16 hours, on an ESR incubator supplied by Kühner AG (Birsfelden, Switzerland). In each case, 250 µl of this preliminary culture are inoculated into 10 ml of production medium (25 g of (NH₄)₂SO₄/l, 2 g of KH₂PO₄/l, 1 g of MgSO₄*7H₂O/l, 0.03 g of FeSO₄*7H₂O/l, 0.018 g of MnSO₄*1H₂O/l, 30 g of CaCO₃/l, 20 g of glucose/l and 50 mg of ampicillin/l), and the latter medium is incubated at 37°C for 48 hours. The formation of L-threonine by the parent strain MG442 is examined in the same way, but without any ampicillin being added to the medium. After the incubation, the optical density (OD) of the culture suspension is determined, at a measurement wavelength of 660 nm, using a Dr. Lange LP2W photometer (Düsseldorf, Germany).

The concentration of L-threonine which has been formed is then determined, in the culture supernatant, which has been sterilized by filtration, by means of ion exchange chromatography using an Eppendorf-BioTronik amino acid analyzer (Hamburg, Germany), and post column reaction involving detection with ninhydrin.

The result of the experiment is shown in Table 1.

**Table 1**

| Strain | OD (660 nm) | L-Threonine g/l |
|---|---|---|
| MG442 | 5.6 | 1.4 |
| MG442/pTrc99A | 3.8 | 1.3 |
| MG442/pTrc99AyodA | 4.7 | 2.1 |

### Brief Description of the Figure:

The length values are to be regarded as being approximate. The abbreviations and terms employed have the following meanings:
• Amp: ampicillin resistance gene
• lacI: gene for the repressor protein of the trc promoter
• Ptrc: trc promoter region, IPTG-inducible
• yodA: coding region of the yodA open reading frame
• 5S: 5S rRNA region
• rrnBT: rRNA terminator region

The abbreviations for the restriction enzymes have the following meanings:
• EcoRI: Escherichia coli RY13 restriction endonuclease
• EcoRV: Escherichia coli B946 restriction endonuclease
• HincII: Haemophilus influenzae R_{c} restriction endonuclease
• NcoI: Rhodococcus sp. ATCC 19070 restriction endonuclease

## Claims

1. A recombinant microorganism of the Enterobacteriaceae family which already produces L-amino acids and which contains an overexpressed yodA ORF, and which produces L-amino acids in an improved manner in comparison to microorganisms which are not recombinant for the yodA ORF, and which do not contain any potentiated yodA ORF.

2. A microorganism of the Enterobacteriaceae family as claimed in claim 1, in which a polynucleotide which corresponds to the yodA ORF and which encodes a polypeptide whose amino acid sequence is at least 90% identical to an amino acid sequence selected from SEQ ID No. 4 and SEQ ID No. 6 is overexpressed.

3. A microorganism as claimed in claim 2,wherein the overexpressed polynucleotide which corresponds to the yodA ORF is selected from the group:
a) polynucleotide having the nucleotide sequence from SEQ ID No. 3 or SEQ ID No. 5;
b) polynucleotide having a nucleotide sequence which corresponds to SEQ ID No. 3 or SEQ ID No. 5 within the limits of the degeneracy of the genetic code;
c) polynucleotide sequence having a sequence which hybridizes, under stringent conditions, with the sequence which is complementary to the sequence SEQ ID No. 3 or SEQ ID No. 5.

4. A microorganism as claimed in claim 2, wherein the polypeptide possesses an amino acid sequence which is at least 95% identical to one of the sequences selected from the group SEQ ID No. 4 and SEQ ID No. 6.

5. A microorganism as claimed in claim 2, wherein the polypeptide has the amino acid sequence which is identical to that from SEQ ID No. 4 or SEQ ID No. 6.

6. A microorganism as claimed in claims 2 to 5, wherein it is prepared by transformation, transduction or conjugation, or a combination of these methods, with a vector which contains the yodA ORF, or an allele of this yodA ORF, and/or a promoter, which potentiates the expression of the yodA ORF or the allele of this yodA ORF.

7. A microorganism as claimed in claim 1 or 6, in which the copy number of the yodA ORF, or of the alleles, is increased by at least 1.

8. A microorganism as claimed in claim 7, wherein the increase in the copy number of the yodA ORF by at least 1 is brought about by integrating the ORF or the alleles into the chromosome of the microorganism.

9. A microorganism as claimed in claim 7, wherein the increase in the copy number of the yodA ORF by at least 1 is achieved by means of an extrachromosomally replicating vector.

10. A microorganism as claimed in claim 1 or 2, wherein, in order to achieve the overexpression,
a) the promoter and regulatory region, or the ribosome binding site upstream of the yodA ORF, is mutated, or
b) expression cassettes or promoters are incorporated upstream of the yodA ORF.

11. A microorganism as claimed in claim 1 or 2, wherein the yodA ORF is under the control of a promoter which potentiates expression of the gene.

12. A microorganism as claimed in claims 1 to 11, wherein the overexpression of the yodA ORF increases the concentration or activity of the yodA gene product (protein) by at least 10%, based on the activity or concentration of the gene product in the microorganism or parent strain which is not recombinant for the yodA ORF.

13. A microorganism as claimed in claim 1 to 12, wherein the microorganism is selected from the genera Escherichia, Erwinia, Providencia and Serratia.

14. A microorganism as claimed in claims 1 to 13, wherein it produces L-threonine.

15. A process for preparing L-amino acids by fermenting recombinant microorganisms of the Enterobacteriaceae family, wherein
a) recombinant microorganisms according to any one of claims 1 to 14 are cultured in a medium under conditions under which the desired L-amino acid is enriched in the medium or in the cells, and
b) the desired L-amino acid is isolated, with, where appropriate, constituents of the fermentation broth, and/or the biomass, in its entirety or in portions (≥ 0 to 100%), remaining in the isolated product or being completely removed.

16. The process as claimed in claim 15, wherein, for the purpose of preparing L-threonine, microorganisms of the Enterobacteriaceae family are fermented in which one or more of the genes selected from the group:
16.1 at least one gene of the thrABC operon encoding aspartate kinase, homoserine dehydrogenase, homoserine kinase and threonine synthase,
16.2 the Corynebacterium glutamicum pyruvate carboxylase-encoding pyc gene,
16.3 the phosphoenolpyruvate synthase-encoding pps gene,
16.4 the phosphoenolpyruvate carboxylase-encoding ppc gene,
16.5 the pntA and pntB genes encoding the pyridine transhydrogenase subunits,
16.6 the rhtB gene encoding the homoserine resistance-mediating protein,
16.7 the rhtC gene encoding the threonine resistance-mediating protein,
16.8 the Corynebacterium glutamicum thrE gene encoding the threonine export carrier protein,
16.9 the glutamate dehydrogenase-encoding gdhA gene,
16.10 the phosphoglucomutase-encoding pgm gene,
16.11 the fructose biphosphate aldolase-encoding fba gene,
16.12 the ptsH gene encoding the phosphohistidine protein hexose phosphotransferase,
16.13 the ptsI gene encoding the enzyme I of the phosphotransferase system,
16.14 the crr gene encoding the glucose-specific IIA component,
16.15 the ptsG gene encoding the glucose-specific IIBC component,
16.16 the lrp gene encoding the leucine regulon regulator,
16.17 the fadR gene encoding the fad regulon regulator,
16.18 the iclR gene encoding the central intermediary metabolism regulator,
16.19 the ahpC gene encoding the small subunit of alkyl hydroperoxide reductase,
16.20 the ahpF gene encoding the large subunit of alkyl hydroperoxide reductase,
16.21 the cysteine synthase A-encoding cysK gene,
16.22 the cysB gene encoding the cys regulon regulator,
16.23 the cysJ gene encoding the NADPH sulfite reductase flavoprotein,
16.24 the cysI gene encoding the NADPH sulfite reductase hemoprotein,
16.25 the cysH gene encoding adenylyl sulfate reductase,
16.26 the rseA gene encoding a membrane protein possessing anti-sigmaE activity,
16.27 the rseC gene encoding a global regulator of the sigmaE factor,
16.28 the sucA gene encoding the decarboxylase subunit of 2-ketoglutarate dehydrogenase,
16.29 the sucB gene encoding the dihydrolipoyl transsuccinase E2 subunit of 2-ketoglutarate dehydrogenase,
16.30 the sucC gene encoding the β subunit of succinyl-CoA synthetase,
16.31 the sucD gene encoding the α subunit of succinyl-CoA synthetase,
16.32 the aceE gene encoding the E1 component of the pyruvate dehydrogenase complex,
16.33 the aceF gene encoding the E2 component of the pyruvate dehydrogenase complex,
16.34 the rseB gene encoding the SigmaE factor activity regulator, and
16.35 the gene product of the Escherichia coli yaaU open reading frame (ORF),
is/are additionally overexpressed at the same time.

17. The process as claimed in claims 15 or 16, wherein, for the purpose of preparing L-threonine, microorganisms of the Enterobacteriaceae family are fermented in which one or more of the genes selected from the group:
17.1 the threonine dehydrogenase-encoding tdh gene,
17.2 the malate dehydrogenase-encoding mdh gene,
17.3 the gene product of the Escherichia coli yjfA open reading frame (ORF),
17.4 the gene product of the Escherichia coli ytfP open reading frame (ORF),
17.5 the phosphoenolpyruvate carboxykinaser-encoding pckA gene
17.6 the pyruvate oxidase-encoding poxB gene,
17.7 the dgsA gene encoding the DgsA regulator of the phosphotransferase system,
17.8 the fructose repressor-encoding fruR gene,
17.9 the Sigma³⁸ factor-encoding rpoS gene, and
17.10 the aspartate ammonium lyase-encoding aspA gene,
is/are additionally attenuated, in particular eliminated, or their expression is reduced, at the same time.

18. The process as claimed in claims 15 to 17, wherein L-amino acids selected from the group L-asparagine, L-serine, L-glutamate, L-glycine, L-alanine, L-cysteine, L-valine, L-methionine, L-proline, L-isoleucine, L-leucine, L-tyrosine, L-phenylalanine, L-histidine, L-lysine, L-tryptophan, L-arginine and L-homoserine are prepared.

19. The process as claimed in claim 18, wherein L-amino acids selected from the group L-isoleucine, L-valine, L-methionine, L-homoserine, L-tryptophan and L-lysine are prepared.

## Patentansprüche

1. Rekombinanter Mikroorganismus der Familie Enterobacteriaceae, der bereits L-Aminosäuren produziert und der einen überexprimierten yodA-ORF enthält und der L-Aminosäuren in verbesserter Weise im Vergleich zu Mikroorganismen, die nicht rekombinant für den yodA-ORF sind und die keinen verstärkten yodA-ORF enthalten, produziert.

2. Mikroorganismus der Familie Enterobacteriaceae nach Anspruch 1, in dem ein Polynukleotid, das dem yodA-ORF entspricht und das ein Polypeptid kodiert, dessen Aminosäuresequenz zu mindestens 90% identisch zu einer Aminosäuresequenz ausgewählt aus SEQ ID Nr. 4 und SEQ ID Nr. 6 ist, überexprimiert wird.

3. Mikroorganismus nach Anspruch 2, wobei das überexprimierte Polynukleotid, das dem yodA-ORF entspricht, aus der folgenden Gruppe ausgewählt ist:
a) Polynukleotid mit der Nukleotidsequenz aus SEQ ID Nr. 3 oder SEQ ID Nr. 5;
b) Polynukleotid mit einer Nukleotidsequenz, die im Rahmen der Degeneriertheit des genetischen Codes SEQ ID Nr. 3 oder SEQ ID Nr. 5 entspricht;
c) Polynukleotidsequenz mit einer Sequenz, die unter stringenten Bedingungen mit der Sequenz, die zu der Sequenz SEQ ID Nr. 3 oder SEQ ID Nr. 5 komplementär ist, hybridisiert.

4. Mikroorganismus nach Anspruch 2, wobei das Polypeptid eine Aminosäuresequenz besitzt, die mindestens zu 95% identisch mit einer der Sequenzen ausgewählt aus der Gruppe SEQ ID Nr. 4 und SEQ ID Nr. 6, ist.

5. Mikroorganismus nach Anspruch 2, wobei das Polypeptid die Aminosäuresequenz hat, die identisch mit der aus SEQ ID Nr. 4 oder SEQ ID Nr. 6 ist.

6. Mikroorganismus nach Anspruch 2 bis 5, wobei er durch Transformation, Transduktion oder Konjugation oder durch eine Kombination dieser Verfahren hergestellt wird, mit einem Vektor, der den yodA-ORF, oder ein Allel dieses yodA-ORF, und/oder einen Promotor enthält, der die Expression des yodA-ORF oder des Allels dieses yodA-ORF verstärkt.

7. Mikroorganismus nach Anspruch 1 oder 6, bei dem die Kopienzahl des yodA-ORF, oder der Allele, um mindestens 1 erhöht wird.

8. Mikroorganismus nach Anspruch 7, wobei die Erhöhung der Kopienzahl des yodA-ORF um mindestens 1 dadurch erzeugt wird, dass das ORF oder die Allele in das Chromosom des Mikroorganismus integriert wird bzw. werden.

9. Mikroorganismus nach Anspruch 7, wobei die Erhöhung der Kopienzahl des yodA-ORF um mindestens 1 mittels eines extrachromosomal replizierenden Vektors erreicht wird.

10. Mikroorganismus nach Anspruch 1 oder 2, wobei, um Überexpression zu erreichen,
a) der Promotor- und Regulationsbereich, oder die Bindungsstelle des Ribosoms stromaufwärts des yodA-ORFs, mutiert ist oder
b) Expressionskassetten oder Promotoren stromaufwärts des yodA-ORF eingebaut sind.

11. Mikroorganismus nach Anspruch 1 oder 2, wobei der yodA-ORF unter der Kontrolle eines Promotors steht, der die Expression des Gens verstärkt.

12. Mikroorganismus nach Anspruch 1 bis 11, wobei die Überexpression des yodA-ORF die Konzentration oder Aktivität des yodA-Genprodukts (-Proteins) um mindestens 10% erhöht, bezogen auf die Aktivität bzw. Konzentration des Genprodukts im Mikroorganismus oder Ausgangsstamm, der für das yodA-ORF nicht rekombinant ist.

13. Mikroorganismus nach Anspruch 1 bis 12, wobei der Mikroorganismus aus den Gattungen Escherichia, Erwinia, Providencia und Serratia ausgewählt ist.

14. Mikroorganismus nach Anspruch 1 bis 13, wobei er L-Threonin produziert.

15. Verfahren zur Herstellung von L-Aminosäuren durch Fermentieren rekombinanter Mikroorganismen der Familie Enterobacteriaceae, wobei
a) rekombinante Mikroorganismen gemäß einem der Ansprüche 1 bis 14 in einem Medium unter Bedingungen kultiviert werden, unter denen die gewünschte L-Aminosäure im Medium oder in den Zellen angereichert wird, und
b) die gewünschte L-Aminosäure isoliert wird, wobei gegebenenfalls Bestandteile der Fermentationsbrühe und/oder die Biomasse vollständig oder anteilsweise (≥ 0 bis 100%) im isolierten Produkt verbleiben oder vollständig abgetrennt werden.

16. Verfahren nach Anspruch 15, wobei zwecks Herstellung von L-Threonin Mikroorganismen der Familie Enterobacteriaceae fermentiert werden, bei denen zusätzlich gleichzeitig ein oder mehrere Gene, ausgewählt aus der Gruppe:
16.1 mindestens ein Gen des Aspartatkinase, Homo-serin-Dehydrogenase, Homoserinkinase und Threoninsynthase codierenden thrABC-Operons,
16.2 das Pyruvatcarboxylase codierende pyc-Gen aus Corynebacterium glutamicum,
16.3 das Phosphoenolpyruvat-Synthase codierende pps-Gen,
16.4 das Phosphoenolpyruvat-Carboxylase codierende ppc-Gen,
16.5 die die Untereinheiten von Pyridin-Trans-hydrogenase codierenden Gene pntA und pntB,
16.6 das das Homoserinresistenz vermittelnde Protein codierende rhtB-Gen,
16.7 das das Threoninresistenz vermittelnde Protein codierende rhtC-Gen,
16.8 das Threoninexport-Carrier-Protein codierende thrE-Gen aus Corynebacterium glutamicum,
16.9 das Glutamat-Dehydrogenase codierende gdhA-Gen,
16.10 das Phosphoglucomutase codierende pgm-Gen,
16.11 das Fructosebiphosphat-Aldolase codierende fba-Gen,
16.12 das die Phosphohistidinprotein-Hexose-Phosphotransferase codierende ptsH-Gen,
16.13 das Enzym I des Phosphotransferase-Systems codierende ptsI-Gen,
16.14 das die glucosespezifische Komponente IIA codierende crr-Gen,
16.15 das die glucosespezifische Komponente IIBC codierende ptsG-Gen,
16.16 das den Regulator des Leucin-Regulons codierende lrp-Gen,
16.17 das den Regulator des fad-Regulons codierende fadR-Gen,
16.18 das den Regulator des zentralen Intermediärstoffwechsels codierende iclR-Gen,
16.19 das die kleine Untereinheit der Alkylhydroperoxid-Reduktase codierende ahpC-Gen,
16.20 das die große Untereinheit der Alkylhydroperoxid-Reduktase codierende ahpF-Gen,
16.21 das Cysteinsynthase A codierende cysK-Gen,
16.22 das den Regulator des cys-Regulons codierende cysB-Gen,
16.23 das das NADPH-Sulfit-Reduktase-Flavoprotein codierende cysJ-Gen,
16.24 das das NADPH-Sulfit-Reduktase-Hämoprotein codierende cysI-Gen,
16.25 das Adenylylsulfat-Reduktase codierende cysH-Gen,
16.26 das ein Membranprotein mit Anti-sigmaE-Aktivität codierende rseA-Gen,
16.27 das eine globalen Regulator des sigmaE-Faktors codierende rseC-Gen,
16.28 das die Decarboxylase-Untereinheit von 2-Ketoglutarat-Dehydrogenase codierende sucA-Gen,
16.29 das die Dihydrolipoyl Transsuccinase-E2-Untereinheit von 2-Ketoglutarat-Dehydrogenase codierende sucB-Gen,
16.30 das die β-Untereinheit von Succinyl-CoA-Synthetase codierende sucC-Gen,
16.31 das die α-Untereinheit von Succinyl-CoA-Synthetase codierende sucD-Gen,
16.32 das die Komponente E1 des Pyruvat-Dehydrogenase-Komplexes codierende aceE-Gen,
16.33 das die Komponente E2 des Pyruvat-Dehydrogenase-Komplexes codierende aceF-Gen,
16.34 das den Regulator der SigmaE-Faktor-Aktivität codierende rseB-Gen, und
16.35 das Genprodukt des offenen yodA-Leserasters (yaaU-ORF) aus Escherichia coli
überexprimiert ist bzw. sind.

17. Verfahren nach Anspruch 15 oder 16, wobei zwecks Herstellung von L-Threonin Mikroorganismen der Familie Enterobacteriaceae fermentiert werden, bei denen zusätzlich gleichzeitig ein oder mehrere Gene, ausgewählt aus der Gruppe:
17.1 das Threonin-Dehydrogenase codierende tdh-Gen,
17.2 das Malat-Dehydrogenase codierende mdh-Gen,
17.3 das Genprodukt des offenen yjfA-Leserasters (yjfA-ORF) aus Escherichia coli,
17.4 das Genprodukt des offenen ytfP-Leserasters (ytfP-ORF) aus Escherichia coli,
17.5 das die Phosphoenolpyruvat-Carboxykinase codierende pckA-Gen,
17.6 das Pyruvatoxidase codierende poxB-Gen,
17.7 das den DgsA-Regulator des Phosphotransferase-Systems codierende dgsA-Gen,
17.8 das den Fructose-Repressor codierende fruR-Gen,
17.9 das den sigma³⁸-Faktor codierende rpoS-Gen und
17.10 das Aspartat-Ammoniumlyase codierende aspA-Gen
abgeschwächt, insbesondere ausgeschaltet ist bzw. sind oder deren Expression vermindert ist.

18. Verfahren nach Anspruch 15 bis 17, wobei L-Aminosäuren ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin hergestellt werden.

19. Verfahren nach Anspruch 18, wobei L-Aminosäuren ausgewählt aus der Gruppe L-Isoleucin, L-Valin, L-Methionin, L-Homoserin, L-Tryptophan und L-Lysin hergestellt werden.

## Revendications

1. Micro-organisme recombinant de la famille des Enterobacteriaceae qui produit déjà des acides aminés L et qui contient un ORF yodA surexprimé, et qui produit des acides aminés L de manière améliorée par comparaison avec des micro-organismes qui sont non recombinants pour l'ORF yodA, et qui ne contiennent pas d'ORF yodA potentialisé.

2. Micro-organisme de la famille des Enterobacteriaceae selon la revendication 1, dans lequel un polynucléotide qui correspond à l'ORF yodA et qui code pour un polypeptide dont la séquence d'acides aminés est à au moins 90 % identique à une séquence d'acides aminés choisie parmi SEQ ID No. 4 et SEQ ID No. 6 est surexprimé.

3. Micro-organisme selon la revendication 2, dans laquelle le polynucléotide surexprimé qui correspond à l'ORF yodA est choisi dans le groupe de :
a) un polynucléotide ayant la séquence nucléotidique de SEQ ID N° 3 ou SEQ ID N° 5 ;
b) un polynucléotide ayant une séquence nucléotidique qui correspond à SEQ ID N° 3 ou SEQ ID N° 5 dans les limites de la dégénérescence du code génétique ;
c) une séquence polynucléotidique ayant une séquence qui s'hybride, dans des conditions stringentes, avec la séquence qui est complémentaire de la séquence SEQ ID N° 3, ou SEQ ID N° 5.

4. Micro-organisme selon la revendication 2, dans lequel le polypeptide possède une séquence d'acides aminés qui est à au moins 95 % identique à une des séquences choisies dans le groupe de SEQ ID N° 4 et SEQ ID N° 6.

5. Micro-organisme selon la revendication 2, dans lequel le polypeptide a la séquence d'acides aminés qui est identique à celle de SEQ ID N° 4 ou SEQ ID N° 6.

6. Micro-organisme selon les revendications 2 à 5, dans lequel il est préparé par transformation, transduction ou conjugaison, ou une combinaison de ces procédés, avec un vecteur qui contient l'ORF yodA, ou un allèle de cet ORF yodA, et/ou un promoteur, qui potentialise l'expression de l'ORF yodA ou de l'allèle de cet ORF yodA.

7. Micro-organisme selon la revendication 1 ou 6, dans lequel le nombre de copies de l'ORF yodA ou des allèles, est augmenté d'au moins 1.

8. Micro-organisme selon la revendication 7, dans lequel l'augmentation du nombre de copies de l'ORF yodA d'au moins 1 est produite par intégration de l'ORF ou des allèles dans le chromosome du micro-organisme.

9. Micro-organisme selon la revendication 7, dans lequel l'augmentation du nombre de copies de l'ORF yodA d'au moins 1 est obtenue au moyen d'un vecteur qui se réplique de manière extrachromosomique.

10. Micro-organisme selon la revendication 1 ou 2, dans lequel, pour obtenir la surexpression a) le promoteur et la région régulatrice, ou le site de liaison ribosomique en amont de l'ORF yodA est muté, ou b) des cassettes d'expression ou des promoteurs sont incorporés en amont de l'ORF yodA.

11. Micro-organisme selon la revendication 1 ou 2, dans lequel l'ORF yodA est sous le contrôle d'un promoteur qui potentialise l'expression du gène.

12. Micro-organisme selon les revendications 1 à 11, dans lequel la surexpression de l'ORF yodA augmente la concentration ou l'activité du produit génique (protéine) yodA d'au moins 10 %, sur la base de l'activité ou de la concentration du produit génique dans le micro-organisme ou la souche parente qui est non recombinante pour l'ORF yodA.

13. Micro-organisme selon la revendication 1 à 12, dans lequel le micro-organisme est choisi parmi les genres Escherichia, Erwinia, Providencia et Serratia.

14. Micro-organisme selon les revendications 1 à 13, dans lequel il produit de la L-thréonine.

15. Procédé pour préparer des acides aminés L par fermentation de micro-organismes recombinants de la famille des Enterobacteriaceae, dans lequel
a) des micro-organismes recombinants selon l'une quelconque des revendications 1 à 14 sont cultivés dans un milieu dans des conditions dans lesquelles l'acide aminé L recherché est enrichi dans le milieu ou dans les cellules, et
b) l'acide aminé L recherché est isolé, des constituants du bouillon de fermentation, et/ou la biomasse, dans son intégralité ou partiellement (≥ 0 à 100 %) restant dans le produit isolé ou étant totalement éliminés, le cas échéant.

16. Procédé selon la revendication 15, dans lequel, en vue de préparer de la L-thréonine, des micro-organismes de la famille des Enterobacteriaceae sont fermentés dans lesquels un ou plusieurs des gènes choisis dans le groupe de :
16.1 au moins un gène de l'opéron thrABC codant pour l'aspartate kinase, l'homosérine déshydrogénase, l'homosérine kinase et la thréonine synthase,
16.2 le gène pyc de Corynebacterium glutamicum codant pour la pyruvate carboxylase,
16.3 le gène pps codant pour la phosphoénolpyruvate synthase,
16.4 le gène ppc codant pour la phosphoénolpyruvate carboxylase,
16.5 les gènes pntA et pntB codant pour les sous-unités de la pyridine transhydrogénase,
16.6 le gène rhtB codant pour la protéine de résistance à l'homosérine,
16.7 le gène rhtC codant pour la protéine de résistance à la thréonine,
16.8 le gène thrE de Corynebacterium glutamicum codant pour la protéine transporteuse d'exportation de thréonine,
16.9 le gène gdhA codant pour la glutamate déshydrogénase,
16.10 le gène pgm codant pour la phosphoglucomutase,
16.11 le gène fba codant pour la fructose biphosphate aldolase,
16.12 le gène ptsH codant pour la phosphohistidine protéine hexose phosphotransférase,
16.13 le gène ptsI codant pour l'enzyme I du système phosphotransférase,
16.14 le gène crr codant pour le composant IIA spécifique du glucose,
16.15 le gène ptsG codant pour le composant IIBC spécifique du glucose,
16.16 le gène lrp codant pour le régulateur du régulon leucine,
16.17 le gène fadR codant pour le régulateur du régulon fad,
16.18 le gène iclR codant pour le régulateur du métabolisme intermédiaire central,
16.19 le gène ahpC codant pour la petite sous-unité de l'alkyl-hydroperoxyde réductase,
16.20 le gène ahpF codant pour la grande sous-unité de l'alkyl-hydroperoxyde réductase,
16.21 le gène cysK codant pour la cystéine synthase A,
16.22 le gène cysB codant pour le régulateur du régulon cys,
16.23 le gène cysJ codant pour le flavoprotéine de NADPH sulfite réductase,
16.24 le gène cysI codant pour l'hémoprotéine de NADPH sulfite réductase,
16.25 le gène cysH codant pour l'adénylyl-sulfate réductase,
16.26 le gène rseA codant pour une protéine membranaire possédant une activité anti-sigmaE,
16.27 le gène rseC codant pour un régulateur global du facteur sigmaE,
16.28 le gène sucA codant pour la sous-unité décarboxylase de la 2-cétoglutarate déshydrogénase,
16.29 le gène sucB codant pour la sous-unité dihydrolipoyl-transsuccinase E2 de la 2-cétoglutarate déshydrogénase,
16.30 le gène sucC codant pour la sous-unité β de la succinyl-CoA synthétase,
16.31 le gène sucD codant pour la sous-unité α de la succinyl-CoA synthétase,
16.32 le gène aceE codant pour le composant E1 du complexe pyruvate déshydrogénase,
16.33 le gène aceF codant pour le composant E2 du complexe pyruvate déshydrogénase,
16.34 le gène rseB codant pour le régulateur de l'activité du facteur SigmaE, et
16.35 le produit génique du cadre ouvert de lecture (ORF) yaaU d'Escherichia coli
est/sont également surexprimés simultanément.

17. Procédé selon la revendication 15 ou 16, dans lequel en vue de préparer de la L-thréonine, des micro-organismes de la famille des Enterobacteriaceae sont fermentés dans lesquels un ou plusieurs des gènes choisis dans le groupe de :
17.1 le gène tdh codant pour la thréonine déshydrogénase,
17.2 le gène mdh codant pour la malate déshydrogénase,
17.3 le produit génique du cadre ouvert de lecture (ORF) yjfA d'Escherichia coli,
17.4 le produit génique du cadre ouvert de lecture (ORF) ytfP d'Escherichia coli,
17.5 le gène pckA codant pour la phosphoénolpyruvate carboxykinase,
17.6 le gène poxB codant pour la pyruvate oxydase,
17.7 gène dgsA codant pour le régulateur DgsA du système phosphotransférase,
17.8 le gène fruR codant pour le répresseur de fructose,
17.9 le gène rpoS codant le facteur sigma³⁸, et,
17.10 le gène aspA codant pour l'aspartate ammonium lyase,est/sont également atténués, en particulier éliminés, ou leur expression est réduite simultanément.

18. Procédé selon les revendications 15 à 17, dans lequel les acides aminés L choisis dans le groupe de L-asparagine, L-sérine, L-glutamate, L-glycine, L-alanine, L-cystéine, L-valine, L-méthionine, L-proline, L-isoleucine, L-leucine, L-tyrosine, L-phénylalanine, L-histidine, L-lysine, L-tryptophane, L-arginine et L-homosérine sont préparés.

19. Procédé selon la revendication 18, dans lequel les acides aminés L choisis dans le groupe de L-isoleucine, L-valine, L-méthionine, L-homosérine, L-tryptophane et L-lysine sont préparés.
